# FASCICULE DE BREVET EUROPEEN

(11) **EP 2 515 529 B1**
(45) Date de publication et mention de la délivrance du brevet: **24.07.2013**
(21) Numéro de dépôt: 12163941.3
(22) Date de dépôt: 12.04.2012
(51) Int. Cl.: H04N 5/247

(54) **Ensemble opératoire comprenant un dispositif de surveillance, utilisation d'un tel ensemble opératoire et installation d'opération**
Operationseinheit, die eine Überwachungsvorrichtung umfasst, Anwendung einer solchen Operationseinheit und Operationsanlage
Surgical assembly comprising a monitoring device, use of such surgical assembly and surgical facility

(30) Priorité: 19.04.2011 FR 1153393
(43) Date de publication de la demande: 24.10.2012
(73) Titulaire: Maquet SAS, 45160 Ardon (FR)
(72) Inventeur: Guilleminot, Bertrand, 41240 Villermain (FR)
(74) Mandataire: Prugneau, Philippe

(56) Documents cités:
- EP-A1- 1 935 365
- EP-A2- 0 672 389
- WO-A1-00/69354
- WO-A1-01/45627
- FR-A1- 2 876 175
- US-A1- 2009 173 846
- US-B2- 7 224 472

## Description

### Domaine technique

La présente invention concerne un ensemble opératoire comprenant un support s'étendant selon un axe central, un dispositif d'éclairage opératoire et un dispositif de surveillance , notamment d'un champ opératoire médical, le dispositif d'éclairage opératoire comprenant un bras de support éclairage monté sur le support en étant mobile en rotation par rapport à un axe de rotation s'étendant parallèlement à l'axe central du support , le dispositif de surveillance comprenant un bras support caméra monté à une première extrémité sur le support en étant perpendiculaire à l'axe central et mobile en rotation par rapport au support selon un axe de rotation s'étendant parallèlement à l'axe central, le bras de support éclairage et le bras support caméra étant indépendants en mouvement de rotation, le dispositif de surveillance comprenant en outre un module de caméra comprenant un support de caméra monté à l'extrémité du bras support caméra opposée à ladite première extrémité et mobile en rotation par rapport au bras support caméra selon un axe de rotation s'étendant parallèlement à l'axe central, et des moyens d'entraînement adaptés pour déplacer le support de caméra par rapport au support et des moyens de commande des moyens d'entraînement.

### Technique antérieure

On connaît des ensembles opératoires comprenant un dispositif de surveillance d'un champ opératoire médical comportant un support et un bras mobile par rapport au support sur lequel est disposée une caméra destinée à filmer le champ opératoire. Cette caméra est disposée sur un bras indépendant qui doit être manipulé par l'équipe médicale au cours de l'intervention. Ce type de dispositif connu ne donne donc qu'un accès limité aux spectateurs situés à distance, et ne permet pas de donner aux spectateurs une vision parfaite de la salle opératoire dans laquelle est disposé ce dispositif de surveillance.

On connaît notamment du document de brevet WO 00/69354 un ensemble opératoire tel que décrit ci-dessus et destiné à permettre à des étudiants d'observer une opération à distance grâce au dispositif de surveillance. Cependant, cet ensemble à caméra unique offre des possibilités d'observation à distance limitées.

On connaît aussi du document de brevet US 7224472 un ensemble opératoire comprenant un support, un dispositif d'éclairage opératoire et un dispositif de surveillance. Cependant, cet ensemble opératoire, destiné à visualiser en 3D des marqueurs, comprend des caméras liées mécaniquement à l'éclairage pour que les signaux optiques provenant des marqueurs détectés par les caméras puissent être évalués dans la zone sans ombre illuminée par le dispositif d'éclairage. De ce fait cet ensemble opératoire ne permet pas une bonne observation à distance.

On connaît enfin du document de brevet EP 0672389 un ensemble opératoire de localisation sur un patient, comprenant un support, un dispositif d'éclairage opératoire et un dispositif de surveillance. Cependant, dans cet ensemble opératoire, le bras de support éclairage et le bras support caméra constituent un seul et même bras articulé, et le bras support de caméra n'est pas entraînable, ce qui limite l'observation à distance.

Le document de brevet WO 01/45627 décrit un ensemble opératoire dont les dispositifs de surveillance et d'éclairage peuvent être manoeuvrés indépendamment.

### Exposé de l'invention

L'invention a pour but de pallier au moins l'un de ces inconvénients et de proposer un ensemble opératoire avec dispositif de surveillance qui permette une bonne visibilité du champ d'opération au cours de l'opération à un spectateur situé à distance. De préférence, le dispositif de surveillance conduit à une gêne faible pour l'équipe médicale pendant l'intervention tout en étant facile à manipuler.

Un autre but est que l'ensemble opératoire avec dispositif de surveillance soit économique.

A cet effet, l'invention a pour objet un ensemble opératoire comprenant un support s'étendant selon un axe central, un dispositif d'éclairage opératoire et un dispositif de surveillance, notamment d'un champ opératoire médical, le dispositif d'éclairage opératoire comprenant un bras de support éclairage monté sur le support en étant mobile en rotation par rapport à un axe de rotation s'étendant parallèlement à l'axe central du support, le dispositif de surveillance comprenant un bras support caméra monté à une première extrémité sur le support en étant perpendiculaire à l'axe central et mobile en rotation par rapport au support selon un axe de rotation s'étendant parallèlement à l'axe central, le bras de support éclairage et le bras support caméra étant indépendants en mouvement de rotation, le dispositif de surveillance comprenant en outre un module de caméra comprenant un support de caméra monté à l'extrémité du bras support caméra opposée à ladite première extrémité et mobile en rotation par rapport au bras support caméra selon un axe de rotation s'étendant parallèlement à l'axe central, et des moyens d'entraînement adaptés pour déplacer le support de caméra par rapport au support et des moyens de commande des moyens d'entraînement, **caractérisé en ce que** le module de caméra comprend deux caméras de surveillance ayant chacune un axe de vision et un sens de vision, en ce que le support de caméra est un bras fixé en son milieu à l'extrémité du bras support caméra et chaque extrémité libre du support de caméra porte une caméra de surveillance de sorte que les caméras de surveillance sont disposées de part et d'autre d'un axe de symétrie passant par le milieu du support de caméra.

Selon des modes particuliers de réalisation, l'ensemble opératoire peut comporter l'une ou plusieurs des caractéristiques suivantes :
- l'ensemble opératoire comprend deux dispositifs d'orientation commandés par les moyens de commande, chaque dispositif étant adapté pour orienter l'une des caméras de surveillance selon au moins un premier axe de rotation par rapport au support de caméra ;
- chaque dispositif d'orientation est adapté pour orienter la caméra de surveillance associée selon un second axe de rotation, perpendiculaire au premier axe de rotation et à l'axe de vision ;
- les moyens de commande comportent un écran adapté pour afficher l'image captée par au moins l'une des caméras de surveillance, et de préférence des deux caméras de surveillance ;
- le dispositif de surveillance comprend en outre des moyens d'entraînement commandés par les moyens de commande et adaptés pour déplacer le bras support caméra par rapport au support et/ou le support de caméra par rapport au bras support caméra ;
- le support de caméra est mobile par rapport au bras support caméra en rotation autour de l'axe selon une plage angulaire donnée ;
- les moyens de commande comprennent une unité de commande adaptée pour reconstituer en trois dimensions la zone captée par les deux caméras de surveillance ;
- l'ensemble opératoire comporte une configuration dans laquelle les axes de vision des caméras de surveillance sont parallèles et les sens de vision sont opposés l'un à l'autre ;
- les moyens de commande comprennent une unité de commande adaptée pour synchroniser les images simultanément envoyées par chaque caméra de surveillance, de manière à fournir une image en relief à un spectateur lorsque les deux caméras de surveillance fixent un même point d'intérêt commun ;
- le dispositif de surveillance définit un plan médian qui s'étend perpendiculairement au plan passant par les deux seconds axes de rotation et parallèlement aux deux seconds axes de rotation et situé à mi-distance entre ces deux seconds axes de rotation, et comporte une configuration dans laquelle les deux caméras de surveillance fixent un même point d'intérêt commun, et le plan médian passe par le point d'intérêt commun.

L'invention a également pour objet l'utilisation d'un ensemble opératoire selon l'invention, dans laquelle les moyens de commande comprennent une configuration dans laquelle chaque dispositif d'orientation est commandable indépendamment l'un de l'autre à l'aide des moyens de commande.

L'invention a également pour objet l'utilisation d'un ensemble opératoire selon l'invention, dans laquelle les axes de vision des caméras de surveillance sont parallèles et les sens de vision sont opposés l'un à l'autre. En variante, le bras support caméra occupe une position fixe.

L'invention a également pour objet l'utilisation d'un ensemble opératoire selon l'invention, dans laquelle le dispositif définit un plan médian qui s'étend perpendiculairement au plan passant par les deux seconds axes de rotation et parallèlement aux deux seconds axes de rotation et situé à mi-distance entre ces deux seconds axes de rotation, et le dispositif comporte une configuration dans laquelle les deux caméras de surveillance fixent un même point d'intérêt commun, et le plan médian passe par le point d'intérêt commun.

L'invention a également pour objet une installation d'opération comprenant une salle opératoire médicale et un ensemble opératoire selon l'invention, dans laquelle les caméras de surveillance sont installées dans la salle opératoire médicale, et notamment dans laquelle au moins une partie des moyens de commande se trouve en dehors de la salle d'opération médicale dans laquelle les caméras de surveillance sont installées.

### Description sommaire des dessins

L'invention sera mieux comprise à la lecture de la description qui va suivre, donnée uniquement à titre d'exemple et faite en se référant aux dessins annexés, sur lesquels :
- la figure 1 est une vue schématique d'une installation d'opération comprenant un dispositif de surveillance selon l'invention muni d'un module de caméra ;

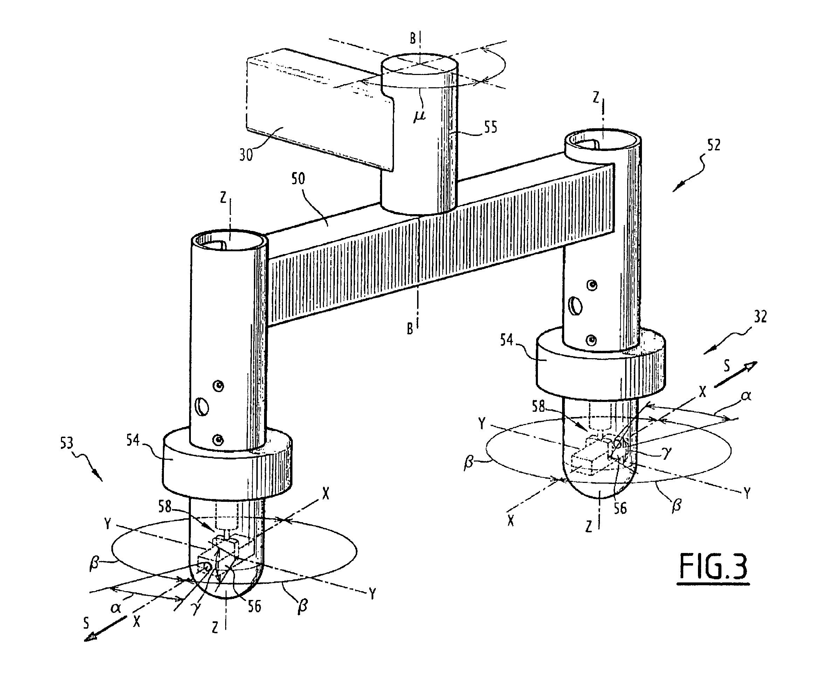

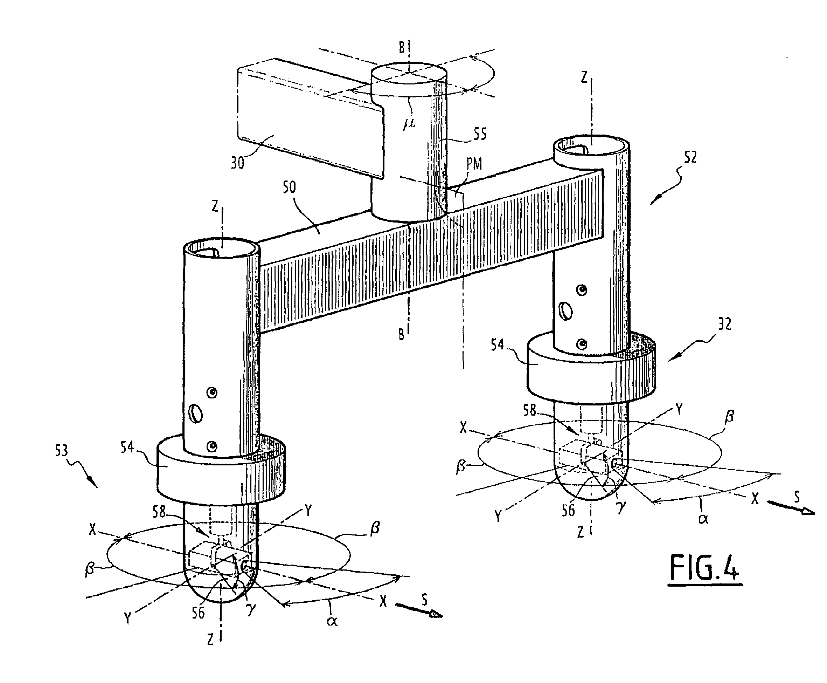

- les figures 2 à 4 sont des vues détaillées du module de caméra représenté à la figure 1 selon, respectivement, une première, une deuxième et une troisième configuration.

### Description des modes de réalisation

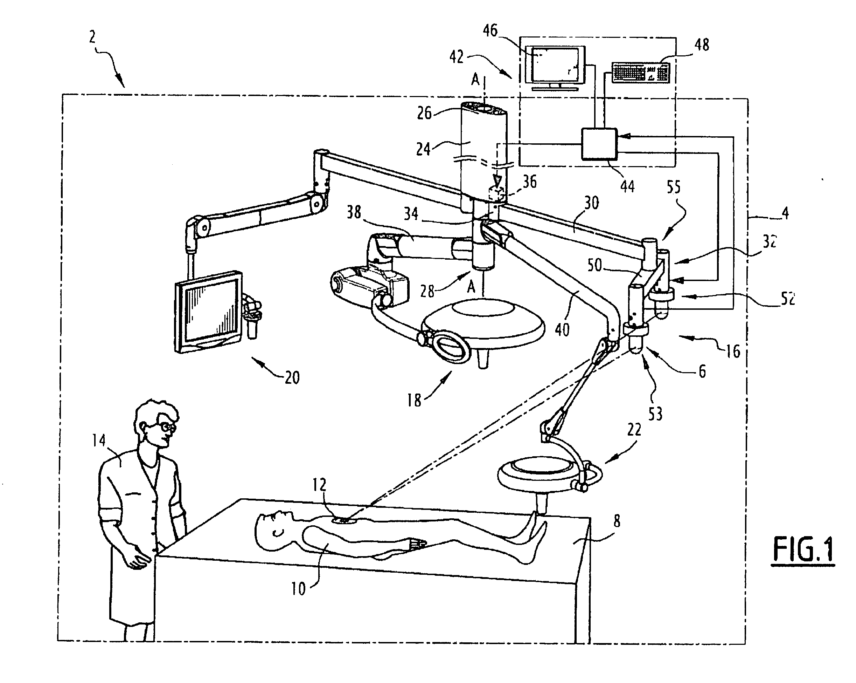

Sur la figure 1 est représentée une installation d'opération selon l'invention, désignée par la référence générale 2. L'installation d'opération 2 comprend une salle opératoire médicale 4 et un ensemble opératoire 6. L'installation d'opération 2 comprend également une table d'opération 8.

Sur la table d'opération 8 se trouve un patient 10 qui définit un champ d'opération médicale 12. Un chirurgien 14 se trouve à proximité du patient 10 de manière à intervenir sur le champ d'opération 12.

L'ensemble opératoire 6 comprend un dispositif de surveillance 16 ainsi qu'un dispositif d'éclairage opératoire principal 18. L'ensemble opératoire 6 comprend également un dispositif d'affichage 20 ainsi qu'un dispositif d'éclairage opératoire secondaire 22.

Le dispositif de surveillance 16 comprend un support 24 qui est un poteau ayant une extrémité de fixation 26 ainsi qu'une extrémité libre 28. Le support 24 s'étend selon un axe central A-A. L'extrémité de fixation 26 est fixée à une structure fixe, telle que le plafond de la salle opératoire médicale 4 ou le mur de cette salle opératoire 4.

Le dispositif de surveillance 16 est muni d'un bras support caméra 30 et d'un module de caméra 32, qui sera détaillé ultérieurement. Le bras support caméra 30 s'étend perpendiculairement au support 24 ou à l'axe central A-A.

Le bras support caméra 30 est mobile par rapport au support 24, de préférence en rotation autour d'un axe de rotation s'étendant parallèlement à l'axe central A-A du support 24. Le bras support caméra 30 est de préférence exclusivement mobile en rotation autour de cet axe de rotation, c'est-à-dire qu'il n'a pas d'autres degrés de liberté de rotation par rapport au support 24.

La liaison mobile en rotation entre le bras support caméra 30 et le support 24 est effectuée au moyen d'une articulation 34. Des moyens d'entraînement 36 adaptés permettent de déplacer le bras support caméra 30 par rapport au support 24. Ces moyens d'entraînement 36 sont par exemple un moteur électrique.

Le dispositif d'éclairage opératoire principal 18 et le dispositif d'éclairage opératoire secondaire 22 comprennent chacun un bras de support éclairage, respectivement 38 et 40, mobile par rapport au support 24, de préférence en rotation autour d'un axe de rotation s'étendant parallèlement à l'axe central A-A du support 24 ou coïncidant avec cet axe central A-A.

Le bras support caméra 30 est disposé au plus près de l'extrémité de fixation 26, le bras de support éclairage 38 supportant le dispositif d'éclairage opératoire principal 18 est disposé au plus près de l'extrémité libre 28, tandis que le bras de support éclairage 40 supportant le dispositif d'éclairage opératoire secondaire 22 est disposé axialement par rapport à l'axe central A-A entre le bras de support éclairage 38 et le bras support caméra 30. Ainsi, le déplacement des dispositifs d'éclairage 18, 22 n'est pas gêné par le bras de support caméra 30.

En outre, le dispositif de surveillance 16 comprend des moyens de commande 42 adaptés pour commander d'une part le module de caméra 32 et d'autre part le déplacement du bras de support caméra 30 en pilotant les moyens d'entraînement 36.

Les moyens de commande 42 sont déportés du support 24 et du bras support caméra 30.

Les moyens de commande 42 comprennent une unité de commande 44. L'unité de commande 44 est relié aux moyens d'entraînement 36 et au module de caméra 32 par des lignes de commande.

Les moyens de commande 42 comprennent également un écran 46 qui est adapté pour afficher ou qui affiche l'image ou les images captée(s) par le module de caméra 32, comme nous le détaillerons ultérieurement. De préférence, l'écran 46 est disposé en dehors de la salle d'opération 4 dans laquelle le module de caméra 32 est installé.

L'unité de commande 44 est relié à l'écran 46 par une connexion de liaison adaptée pour transmettre les images.

Les moyens de commande 42 comprennent également un périphérique d'entrée 48, par exemple un clavier, adapté pour saisir des commandes afin de commander le module de caméra 32 et/ou le déplacement du bras support caméra 30. De préférence, le périphérique d'entrée 48 est disposé en dehors de la salle d'opération 4 dans laquelle le module de caméra 32 est installé. L'unité de commande 44 est reliée au périphérique d'entrée 48 par une connexion adaptée pour transmettre les commandes saisies via le périphérique d'entrée à l'unité de commande 44.

Nous allons à partir de ce point décrire le module de caméra 32.

Le module de caméra 32 est disposé sur une extrémité du bras support caméra 30, ladite extrémité est opposée à celle assurant la liaison avec le support 24.

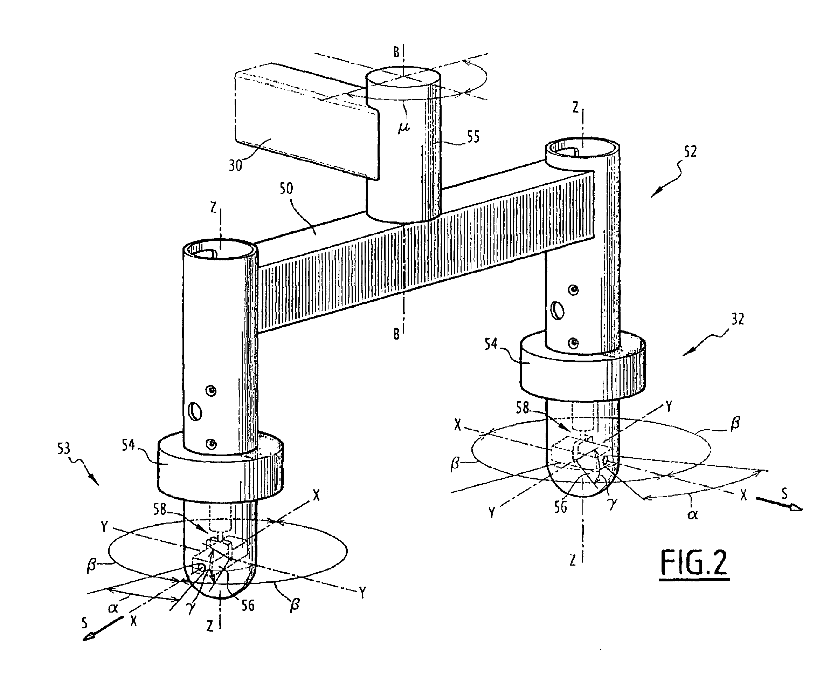

Comme ceci est visible sur la figure 2, le module caméra 32 comprend un support de caméra, en l'occurrence un bras 50 fixé en son milieu à l'extrémité du bras support caméra 30. Chaque extrémité libre du bras 50 porte un dispositif de vision respectivement 52 et 53. Les dispositifs de vison 52 et 53 sont identiques, un seul sera décrit par la suite.

Chaque dispositif de vision 52, 53 comprend un boîtier 54 fixé à une extrémité du bras 50, une caméra de surveillance 56 et un dispositif d'orientation 58 adapté pour déplacer la caméra de surveillance 56 par rapport au boîtier 54 et en conséquence par rapport au bras 50.

Le bras 50 est mobile par rapport au bras support caméra 30, de préférence en rotation autour d'un axe B-B s'étendant parallèlement à l'axe central A-A du support 24 et donc perpendiculairement au bras support caméra 30. Le bras 50 est de préférence exclusivement mobile en rotation autour de cet axe B-B, c'est-à-dire qu'il n'a pas d'autres degrés de liberté de rotation par rapport au bras support caméra 30. La liaison mobile en rotation entre le bras 50 et le bras support caméra 30 est effectué au moyen d'une articulation 55. Un moteur d'entraînement adapté permet le déplacement du bras 50 par rapport au bras support caméra 30. Le bras 50 est articulé autour de l'axe B-B dans sa partie courante, de préférence à mi-chemin de ses extrémités.

Une position neutre du bras 50 est définie, dans laquelle le bras 50 s'étend perpendiculairement au bras support caméra 30. Le bras 50 est ainsi mobile autour d'une plage angulaire µ limitée à +90°/-90° autour de sa position neutre.

Chaque caméra de surveillance 56 est disposée de part et d'autre d'un axe de symétrie passant par le milieu du bras 50.

Chaque caméra de surveillance 56 définit un axe de vision X-X et un sens de vision S qui coïncide avec l'axe de vision X-X.

Le dispositif d'orientation 58 est adapté pour orienter la caméra de surveillance 56 selon un premier axe de rotation Y-Y. A cet effet, le dispositif d'orientation 58 comprend un moteur d'entraînement selon l'axe Y-Y. Le premier axe de rotation Y-Y est disposé perpendiculairement à l'axe de vision X-X. Le dispositif d'orientation 58 est également adapté pour orienter la caméra de surveillance selon un second axe de rotation Z-Z, qui est perpendiculaire au premier axe de rotation Y-Y et à l'axe de vision X-X. A cet effet, le dispositif d'orientation 58 comprend un moteur d'entraînement selon l'axe Z-Z. Les axes X-X, Y-Y et Z-Z définissent un point commun d'intersection.

La position neutre du bras 50 ou plus généralement du support de caméra, est définie par la position du support de caméra, dans laquelle le plan passant par les deux seconds axes de rotation Z-Z appartenant aux dispositifs de vison 52 et 53, s'étend perpendiculairement au bras 30.

Le dispositif d'orientation 58 définit une position neutre de la caméra de surveillance 56. Le dispositif d'orientation 58 est adapté pour tourner la caméra de surveillance autour d'une plage angulaire β limitée à +180°/-180° autour de la position neutre par rapport à l'axe Y-Y.

De même, le dispositif d'orientation 58 est adapté pour tourner la caméra de surveillance autour d'une plage angulaire γ limitée à +45°/-45° autour de la position neutre par rapport à l'axe Z-Z.

Chaque caméra de surveillance 56 a un angle de vision α compris entre 30°et 90° et de préférence compris entre 40° et 60°. Egalement, le zoom maximal de la caméra de surveillance 56 indiqué en distance focale est compris entre 3mm et 180mm et notamment compris entre 3,4 mm et 120 mm.

De préférence, la caméra de surveillance 56 est une caméra IP, c'est-à-dire une caméra qui est adaptée pour transmettre des données ou l'image captée via une liaison Internet protocole/TCP. Egalement, la caméra de surveillance 56 est pilotable par une liaison IP/TCP. En particulier, le zoom de la caméra de surveillance 56 est réglable et pilotable. De même, les moteurs d'entraînement selon les axes Y-Y et Z-Z sont de préférence pilotables par la liaison IP/TCP.

Par ailleurs, le module de caméra 32 peut comporter un microphone (non représenté) adapté pour capter le son de la salle opératoire médicale. Ce microphone est de préférence relié aux moyens de commande 42 et les moyens de commande 42 sont adaptés pour transmettre le son capté à un haut parleur. Les moyens de commande peuvent à cet effet également transmettre le son par une liaison IP/TCP.

Selon une première configuration et un premier mode d'utilisation de ce dispositif de surveillance, les deux caméras de surveillance 56, appartenant chacune à un dispositif de vision, respectivement 52, 53, sont utilisées indépendamment l'une de l'autre et permettent ainsi au spectateur disposé face à l'écran 46 d'observer deux points de vue différents de la salle opératoire médicale 4.

Dans cette configuration et dans ce mode d'utilisation, les moyens de commande 42 sont adaptés pour commander le déplacement du bras support caméra 30 et le déplacement du bras 50 supportant les dispositifs de vision 52, 53. Dans cette configuration, l'orientation et le zoom sont commandés indépendamment d'une caméra de surveillance 56 à l'autre caméra de surveillance 56.

En d'autres termes, une entrée de commande faite par le périphérique d'entrée 48 et associée à l'une des caméras de surveillance ne prend effet que pour cette caméra, et n'entraîne pas une commande de l'autre des caméras.

Dans ce mode, l'écran 46 affiche deux images indépendantes, dont chacune représente l'image délivrée respectivement par l'une des caméras de surveillance 56. Deux points de vue différents de la salle peuvent donc être surveillés simultanément par le module caméra 32.

Ainsi, le spectateur peut visionner au mieux le champ d'opération médicale 12 selon deux points de vue différents.

Ces deux points de vue peuvent notamment être intéressants dans le cas où le champ de vision de l'une des caméras est masqué par une personne présente dans la salle opératoire médicale 4.

Dans une seconde configuration et selon un second mode d'utilisation, les deux caméras de surveillance 56 appartenant chacune à un dispositif de vision, respectivement 52, 53, sont orientées dos-à-dos de manière à filmer chacune des parties opposées de la salle opératoire médicale 4.

Dans cette seconde configuration, les axes de vision X-X des deux caméras de surveillance 56 sont parallèles et les sens de vision sont dirigés à l'opposé l'un de l'autre. De préférence, les deux axes de vision X-X coïncident et se trouvent dans le plan passant par les deux axes de rotation Z-Z.

Dans ce mode d'utilisation, seul le bras 50 est mobile par rapport au bras support caméra 30 autour de la plage angulaire µ.

De préférence dans cette configuration, la position du bras support caméra 30 est fixe, c'est-à-dire qu'une fois déterminée, la position du bras support caméra 30 n'est plus modifiée. De même, une fois la mise au point effectuée, le dispositif d'orientation 58 n'est plus modifié, ce qui signifie également que la distance focale reste constante lors de la mise en oeuvre de cette seconde configuration.

Lors de la mise au point, les caméras de surveillance 56 sont réglées de manière à filmer des directions opposées. A cet effet, une fois l'une des caméras de surveillance 56 positionnée, les moyens de commande 42 positionnent correctement l'autre caméra de surveillance 56 dans la position souhaitée.

En outre, pour une utilisation efficace de cette seconde configuration, l'unité de commande 44 possède un logiciel adapté pour reconstituer virtuellement la salle opératoire médicale 4 en trois dimensions, à partir des images recueillies par les deux caméras de surveillance 56 et générer une image en deux dimensions.

Grâce à ce mode d'utilisation, le spectateur peut alors évoluer virtuellement dans la salle opératoire médicale 4 sans être présent physiquement. Grâce au périphérique d'entrée, le spectateur peut se déplacer dans la salle opératoire médicale 4 reconstituée.

Selon une troisième configuration et un troisième mode d'utilisation, les caméras de surveillance 56 sont utilisées en mode « vision stéréoscopique ». Dans ce mode d'utilisation, les deux caméras de surveillance 56 fixent un point d'intérêt commun, qui peut notamment être le champ d'opération 12 ou une partie de ce champ.

Afin de générer une image stéréoscopique au spectateur, les caméras de surveillance 56 doivent prendre une position de stéréoscopie par rapport au point d'intérêt. Le dispositif définit un plan médian PM s'étendant perpendiculairement à un plan passant par les axes Z-Z, parallèlement à ces deux axes Z-Z et situé à mi-distance entre ces deux axes Z-Z. Dans la position de stéréoscopie, le plan médian PM passe par le point d'intérêt commun. Cette condition évite les aberrations dans l'image stéréoscopique restituée.

Dans cette configuration, l'utilisateur définit à l'aide du périphérique d'entrée 48 le point d'intérêt à observer. L'unité de commande 44 pilote alors le bras support caméra 30 et/ou le bras 50 et/ou le dispositif d'orientation 58 des caméras de surveillance 56 de manière à ce qu'elles soient positionnées dans le positionnement de stéréoscopie comme mentionné ci-dessus.

L'utilisateur peut toujours commander le zoom des caméras afin d'obtenir la vision qu'il souhaite, rapprochée ou de foin, de ce point d'intérêt.

L'unité de commande 44 est adaptée pour synchroniser les images reçues de chaque caméra de surveillance 56 de manière à restituer sur l'écran 46 une seule image stéréoscopique visualisant le point d'intérêt en relief. A cet effet, l'unité de commande 44 est munie d'un logiciel de stéréoscopie qui est adapté pour combiner les images des caméras de surveillance simultanément reçues.

L'image stéréoscopique peut être une image anaglyphe, une image polarisée, une image à décalage dans le temps ou toute autre image stéréoscopique.

Les trois modes de configuration et d'utilisation peuvent être utilisés par un seul dispositif de surveillance et implémenté dans un seul dispositif de surveillance, le spectateur sélectionnant la configuration et le mode dans lequel il souhaite utiliser le dispositif de surveillance.

En variante, le support de caméra 50 du dispositif de surveillance selon l'invention peut être fixé directement sur le support 24. Dans ce cas, il est fixé de préférence au voisinage de l'extrémité libre 28 de ce support 24. Dans cet exemple de réalisation, le dispositif de surveillance ne comporte pas le bras support caméra 30 décrit ci-dessus. Les trois modes de configuration et d'utilisation décrits ci-dessus peuvent également être mis oeuvre par cette variante.

Dans ce cas, le support de caméra 50 est également mobile en rotation par rapport au support 24 et ceci de préférence autour de l'axe de rotation B-B qui est parallèle à l'axe central A-A. Plus particulièrement, l'axe de rotation B-B coïncide avec l'axe central A-A. La mobilité en rotation par rapport au support est de nouveau obtenue par une articulation 55.

Le dispositif de surveillance selon l'invention est donc adapté pour une inspection par un spectateur du champ d'opération médicale à distance et pour un déplacement d'une manière autonome du module de caméra 32 ou du bras support caméra 30 sans la nécessité de l'intervention de l'équipe chirurgicale, qui peut se concentrer sur l'intervention chirurgicale.

## Revendications

1. Ensemble opératoire comprenant un support (24) s'étendant selon un axe central (A-A), un dispositif d'éclairage opératoire (18, 22) et un dispositif de surveillance (16), notamment d'un champ opératoire médical, le dispositif d'éclairage opératoire (18, 22) comprenant un bras de support éclairage (38, 40) monté sur le support (24) en étant mobile en rotation par rapport à un axe de rotation s'étendant parallèlement à l'axe central (A-A) du support (24), le dispositif de surveillance (16) comprenant un bras support caméra (30) monté à une première extrémité sur le support (24) en étant perpendiculaire à l'axe central (A-A) et mobile en rotation par rapport au support (24) selon un axe de rotation s'étendant parallèlement à l'axe central (A-A), le bras de support éclairage (38, 40) et le bras support caméra (30) étant indépendants en mouvement de rotation, le dispositif de surveillance (16) comprenant en outre un module de caméra (32) comprenant un support de caméra (50) monté à l'extrémité du bras support caméra (30) opposée à ladite première extrémité et mobile en rotation par rapport au bras support caméra (30) selon un axe de rotation (B-B) s'étendant parallèlement à l'axe central (A-A), et des moyens d'entraînement adaptés pour déplacer le support de caméra (50) par rapport au support (24) et des moyens de commande (42) des moyens d'entraînement, **caractérisé en ce que** le module de caméra (32) comprend deux caméras de surveillance (56) ayant chacune un axe de vision (X-X) et un sens de vision (S), **en ce que** le support de caméra (50) est un bras fixé en son milieu à l'extrémité du bras support caméra (30) et chaque extrémité libre du support de caméra (50) porte une caméra de surveillance (56) de sorte que les caméras de surveillance (56) sont disposées de part et d'autre d'un axe de symétrie passant par le milieu du support de caméra (50).

2. Ensemble opératoire selon la revendication 1, **caractérisé en ce qu'**il comprend deux dispositifs d'orientation (58) commandés par les moyens de commande (42), chaque dispositif étant adapté pour orienter l'une des caméras de surveillance (56) selon au moins un premier axe de rotation (Y-Y) par rapport au support de caméra (50).

3. Ensemble opératoire selon la revendication 2, **caractérisé en ce que** chaque dispositif d'orientation (58) est adapté pour orienter la caméra de surveillance associée selon un second axe de rotation (Z-Z), perpendiculaire au premier axe de rotation (Y-Y) et à l'axe de vision (X-X).

4. Ensemble opératoire selon l'une quelconque des revendications précédentes, **caractérisé en ce que** les moyens de commande (42) comportent un écran (46) adapté pour afficher l'image captée par au moins l'une des caméras de surveillance (56), et de préférence des deux caméras de surveillance (56).

5. Ensemble opératoire selon l'une quelconque des revendications précédentes, **caractérisé en ce que** le dispositif de surveillance (16) comprenant en outre des moyens d'entraînement commandés par les moyens de commande (42) et adaptés pour déplacer le bras support caméra (30) par rapport au support (24) et/ou le support de caméra (50) par rapport au bras support caméra (30).

6. Ensemble opératoire selon l'une quelconque des revendications précédentes, **caractérisé en ce que** le support de caméra (50) est mobile par rapport au bras support caméra (30) en rotation autour de l'axe (B-B) selon une plage angulaire donnée (µ).

7. Ensemble opératoire selon l'une quelconque des revendications précédentes, **caractérisé en ce que** les moyens de commande (42) comprennent une unité de commande (44) adaptée pour reconstituer en trois dimensions la zone captée par les deux caméras de surveillance (56).

8. Ensemble opératoire selon la revendication 7, **caractérisé en ce qu'**il comporte une configuration dans laquelle les axes de vision (X-X) des caméras de surveillance (56) sont parallèles et les sens de vision (S) sont opposés l'un à l'autre.

9. Ensemble opératoire selon l'une quelconque des revendications précédentes, **caractérisé en ce que** les moyens de commande (42) comprennent une unité de commande (44) adaptée pour synchroniser les images simultanément envoyées par chaque caméra de surveillance (56), de manière à fournir une image en relief à un spectateur lorsque les deux caméras de surveillance (56) fixent un même point d'intérêt commun.

10. Ensemble opératoire selon la revendication 3 et la revendication 9, **caractérisé en ce que** le dispositif de surveillance (16) définit un plan médian (PM) qui s'étend perpendiculairement au plan passant par les deux seconds axes de rotation (Z-Z) et parallèlement aux deux seconds axes de rotation (Z-Z) et situé à mi-distance entre ces deux seconds axes de rotation (Z-Z), et ce qu'il comporte une configuration dans laquelle les deux caméras de surveillance (56) fixent un même point d'intérêt commun, et le plan médian passe par le point d'intérêt commun.

11. Utilisation d'un ensemble opératoire selon la revendication 2 combinée avec l'une quelconque des revendications précédentes, dans laquelle les moyens de commande (42) comprennent une configuration dans laquelle chaque dispositif d'orientation (58) est commandable indépendamment l'un de l'autre à l'aide des moyens de commande (42).

12. Utilisation d'un ensemble opératoire selon les revendications 7 ou 8, dans laquelle les axes de vision (X-X) des caméras de surveillance (56) sont parallèles et les sens de vision (S) sont opposés l'un à l'autre.

13. Utilisation d'un ensemble opératoire selon la revendication 12, dans laquelle le bras support caméra (30) occupe une position fixe.

14. Utilisation d'un ensemble opératoire selon la revendication 3 et une des revendications 9 ou 10, dans laquelle le dispositif définit un plan médian (PM) qui s'étend perpendiculairement au plan passant par les deux seconds axes de rotation (Z-Z) et parallèlement aux deux seconds axes de rotation (Z-Z) et situé à mi-distance entre ces deux seconds axes de rotation (Z-Z), et le dispositif comporte une configuration dans laquelle les deux caméras de surveillance (56) fixent un même point d'intérêt commun, et le plan médian passe par le point d'intérêt commun.

15. Installation d'opération (2) comprenant une salle opératoire médicale (4) et un ensemble opératoire selon au moins l'une quelconque des revendications 1 à 10, dans laquelle les caméras de surveillance (56) sont installées dans la salle opératoire médicale (4), et notamment dans laquelle au moins une partie des moyens de commande (42) se trouve en dehors de la salle d'opération médicale (4) dans laquelle les caméras de surveillance (56) sont installées.

## Patentansprüche

1. Operationseinheit, umfassend eine Stütze (24), die sich entlang einer Mittelachse (A-A) erstreckt, eine Operationsbeleuchtungsvorrichtung (18, 22) und eine Überwachungsvorrichtung (16), insbesondere eines medizinischen Operationsfeldes, wobei die Operationsbeleuchtungsvorrichtung (18, 22) einen Beleuchtungsstützarm (38, 40) umfasst, der auf der Stütze (24) montiert und drehbar in Bezug zu einer Drehachse beweglich ist, die sich parallel zur Mittelachse (A-A) der Stütze (24) erstreckt, wobei die Überwachungsvorrichtung (16) einen Kamerastützarm (30) umfasst, der an einem ersten Ende auf der Stütze (24) montiert ist, wobei er zur Mittelachse (A-A) senkrecht und drehbar in Bezug zur Stütze (24) entlang einer Drehachse, die sich parallel zur Mittelachse (A-A) erstreckt, beweglich ist, wobei der Beleuchtungsstützarm (38, 40) und der Kamerastützarm (30) in der Drehbewegung unabhängig sind, wobei die Überwachungsvorrichtung (16) ferner ein Kameramodul (32) aufweist, umfassend eine Kamerastütze (50), die am Ende des Kamerastützarms (30) gegenüber dem ersten Ende montiert und drehbar in Bezug zum Kamerastützarm (30) entlang einer Drehachse (B-B), die sich parallel zur Mittelachse (A-A) erstreckt, beweglich ist, und Antriebsmittel, die geeignet sind, die Kamerastütze (50) in Bezug zur Stütze (24) zu bewegen, und Steuermittel (42) für die Antriebsmittel, **dadurch gekennzeichnet, dass** das Kameramodul (32) zwei Überwachungskameras (56) umfasst, die jeweils eine Sichtachse (X-X) und eine Sichtrichtung (S) aufweisen, dass die Kamerastütze (50) ein Arm ist, der in seiner Mitte am Ende des Kamerastützarms (30) befestigt ist, und jedes freie Ende der Kamerastütze (50), eine Überwachungskamera (56) trägt, so dass die Überwachungskameras (56) beiderseits einer Symmetrieachse angeordnet sind, die durch die Mitte der Kamerastütze (50) verläuft.

2. Operationseinheit nach Anspruch 1, **dadurch gekennzeichnet, dass** sie zwei Ausrichtungsvorrichtungen (58) umfasst, die von den Steuermitteln (42) gesteuert werden, wobei jede Vorrichtung dazu geeignet ist, eine der Überwachungskameras (56) gemäß mindestens einer ersten Drehachse (Y-Y) in Bezug zur Kamerastütze (50) auszurichten.

3. Operationseinheit nach Anspruch 2, **dadurch gekennzeichnet, dass** jede Ausrichtungsvorrichtung (58) dazu geeignet ist, die zugehörige Überwachungskamera gemäß einer zweiten Drehachse (Z-Z) senkrecht auf die erste Drehachse (Y-Y) und die Sichtachse (X-X) auszurichten.

4. Operationseinheit nach einem der vorhergehenden Ansprüche, **dadurch gekennzeichnet, dass** die Steuermittel (42) einen Bildschirm (46) umfassen, der dazu geeignet ist, das von mindestens einer der Überwachungskameras (56) und vorzugsweise von den beiden Überwachungskameras (56) aufgenommene Bild anzuzeigen.

5. Operationseinheit nach einem der vorhergehenden Ansprüche, **dadurch gekennzeichnet, dass** die Überwachungseinheit (16) ferner Antriebsmittel umfasst, die von den Steuermitteln (42) gesteuert werden und dazu geeignet sind, den Kamerastützarm (30) in Bezug zur Stütze (24) und/oder die Kamerastütze (50) in Bezug zum Kamerastützarm (30) zu bewegen.

6. Operationseinheit nach einem der vorhergehenden Ansprüche, **dadurch gekennzeichnet, dass** die Kamerastütze (50) in Bezug zum Kamerastützarm (30) in Drehung um die Achse (B-B) entlang eines gegebenen Winkelbereichs (µ) beweglich ist.

7. Operationseinheit nach einem der vorhergehenden Ansprüche, **dadurch gekennzeichnet, dass** die Steuermittel (42) eine Steuereinheit (44) umfassen, die dazu geeignet ist, die von den beiden Überwachungskameras (56) aufgenommene Zone dreidimensional wiederzugeben.

8. Operationseinheit nach Anspruch 7, **dadurch gekennzeichnet, dass** sie eine Ausführung umfasst, bei der die Sichtachsen (X-X) der Überwachungskameras (56) parallel sind und die Sichtrichtungen (S) zueinander entgegengesetzt sind.

9. Operationseinheit nach einem der vorhergehenden Ansprüche, **dadurch gekennzeichnet, dass** die Steuermittel (42) eine Steuereinheit (44) umfassen, die dazu geeignet ist, die gleichzeitig von jeder Überwachungskamera (56) gesandten Bilder zu synchronisieren, um einem Betrachter ein Reliefbild zu liefern, wenn die beiden Überwachungskameras (56) einen selben Punkt von gemeinsamem Interesse fixieren.

10. Operationseinheit nach Anspruch 3 und Anspruch 9, **dadurch gekennzeichnet, dass** die Überwachungsvorrichtung (16) eine Mittelebene (PM) definiert, die sich senkrecht auf die Ebene, die durch die beiden zweiten Drehachsen (Z-Z) verläuft, und parallel zu den beiden zweiten Drehachsen (Z-Z) erstreckt und auf halbem Abstand zwischen diesen beiden zweiten Drehachsen (Z-Z) angeordnet ist, und dass sie eine Ausführung umfasst, bei der die beiden Überwachungskameras (56) einen selben Punkt von gemeinsamem Interesse fixieren, und dass die Mittelebene durch den Punkt von gemeinsamem Interesse verläuft.

11. Verwendung einer Operationseinheit nach Anspruch 2 in Kombination mit einem der vorhergehenden Ansprüche, bei der die Steuermittel (42) eine Ausführung umfassen, bei der jede Ausrichtungsvorrichtung (58) unabhängig von der anderen mit Hilfe der Steuermittel (42) steuerbar ist.

12. Verwendung einer Operationseinheit nach den Ansprüchen 7 oder 8, bei der die Sichtachsen (X-X) der Überwachungskameras (56) parallel sind und die Sichtrichtungen (S) zueinander entgegengesetzt sind.

13. Verwendung einer Operationseinheit nach Anspruch 12, bei der der Kamerastützarm (30) eine feste Position einnimmt.

14. Verwendung einer Operationseinheit nach Anspruch 3 und einem der Ansprüche 9 oder 10, bei der die Vorrichtung eine Mittelebene (PM) definiert, die sich senkrecht auf die Ebene, die durch die beiden zweiten Drehachsen (Z-Z) verläuft, und parallel zu den beiden zweiten Drehachsen (Z-Z) erstreckt und auf halbem Abstand zwischen diesen zweiten Drehachsen (Z-Z) angeordnet ist, und bei der die Vorrichtung eine Ausführung umfasst, bei der die beiden Überwachungskameras (56) einen selben Punkt von gemeinsamem Interesse fixieren, wobei die Mittelebene durch den Punkt von gemeinsamem Interesse verläuft.

15. Operationsanlage (2), umfassend einen medizinischen Operationssaal (4) und eine Operationseinheit nach mindestens einem der Ansprüche 1 bis 10, bei der die Überwachungskameras (56) in dem medizinischen Operationssaal (4) installiert sind, und insbesondere bei der sich mindestens ein Teil der Steuermittel (42) außerhalb des medizinischen Operationssaals (4) befindet, in dem die Überwachungskameras (56) installiert sind.

## Claims

1. An operating assembly comprising a support (24) extending along a central axis (A-A), an operating lighting apparatus (18, 22), and an observation apparatus (16), in particular for observing a medical operating field, the operating lighting apparatus (18, 22) having a lighting support arm (38, 40) mounted on the support (24) to be pivotally movable about a pivot axis extending parallel to the central axis (A-A) of the support (24), the observation apparatus (16) comprising a camera support arm (30) mounted at a first end on the support (24) to be perpendicular to the central axis (A-A) and to be pivotally moveable relative to the support (24) about a pivot axis extending parallel to the central axis (A-A), the lighting support arm (38, 40) and the camera support arm (30) being independent in pivotal movement, the observation apparatus (16) further comprising a camera module (32) comprising a camera support (50) mounted on the end of the camera support arm (30) that is opposite from said first end and to be pivotally movable relative to the camera support arm (30) about a pivot axis (B-B) extending parallel to the central axis (A-A), and drive means adapted to move the camera support (50) relative to the support (24), and control means (42) for controlling the drive means, said operating assembly being **characterized in that** the camera module (32) comprises two observation cameras (56), each of which has a vision axis (X-X) and a vision direction (S), and **in that** the camera support (50) is an arm fastened in its middle to the end of the camera support arm (30) and each free end of the camera support (50) carries an observation camera (56) so that the observation cameras (56) are disposed on either side of an axis of symmetry passing through the middle of the camera support (50).

2. An operating assembly according to claim 1, **characterized in that** it further comprises two steering apparatuses (58) controlled by the control means (42), each apparatus being adapted to steer one of the observation cameras (56) about at least a first pivot axis (Y-Y) relative to the camera support (50).

3. An operating assembly according to claim 2, **characterized in that** each steering apparatus (58) is adapted to steer the observation camera about a second pivot axis (Z-Z) perpendicular to the first pivot axis (Y-Y) and to the vision axis (X-X).

4. An operating assembly according to any preceding claim, **characterized in that** the control means (42) include a screen (46) adapted to display the image sensed by at least one of the observation cameras (56), and preferably by both of the observation cameras (56).

5. An operating assembly according to any preceding claim, **characterized in that** the observation apparatus (16) further comprises drive means controlled by the control means (42) and adapted to move the camera support arm (30) relative to the support (24) and/or to move the camera support (50) relative to the camera support arm (30).

6. An operating assembly according to any preceding claim, **characterized in that** the camera support (50) is mounted to move relative to the camera support arm (30) in pivoting about the axis (B-B) through a given angular range (µ).

7. An operating assembly according to any preceding claim, **characterized in that** the control means (42) further comprise a control unit (44) adapted to reconstruct in three dimensions the zone sensed by the two observation cameras (56).

8. An operating assembly according to claim 7, **characterized in that** it has a configuration in which the vision axes (X-X) of the observation cameras (56) are parallel to each other and the vision directions (S) are opposite to each other.

9. An operating assembly according to any preceding claim, **characterized in that** the control means (42) further comprise a control unit (44) adapted to synchronize the images simultaneously sent by each observation camera (56), so as to deliver an image in 3D to a spectator when the two observation cameras (56) are observing the same common point of interest.

10. An operating assembly according to claim 3 and claim 9, **characterized in that** the observation apparatus (16) defines a midplane (PM) that extends perpendicularly to the plane containing the two second pivot axes (Z-Z) and parallel to the two second pivot axes (Z-Z), and that is situated half-way between said two second pivot axes (Z-Z), and **in that** said observation apparatus has a configuration in which the two observation cameras (56) are observing the same common point of interest, and the midplane contains the common point of interest.

11. The use of an operating assembly according to claim 2, combined with any preceding claim, wherein the control means (42) have a configuration in which each steering apparatus (58) is controllable independently from each other by the control means (42).

12. The use of an operating assembly according to claim 7 or claim 8, wherein the vision axes (X-X) of the observation cameras (56) are parallel to each other and the vision directions (S) are opposite to each other.

13. The use of an operating assembly according to claim 12, wherein the camera support arm (30) occupies a stationary position.

14. The use of an operating assembly according to claim 3 and to claim 9 or claim 10, wherein the apparatus defines a midplane (PM) that extends perpendicularly to the plane containing the two second pivot axes (Z-Z) and parallel to the two second pivot axes (Z-Z), and that is situated half-way between said two second pivot axes (Z-Z), and the apparatus has a configuration in which the two observation cameras (56) are observing the same common point of interest, and the midplane contains the common point of interest.

15. An operating facility (2) comprising a medical operating theater (4) and an operating assembly according to any one of claims 1 to 10, wherein the observation cameras (56) are installed in the medical operating theater (4), and in particular wherein at least some of the control means (42) are situated outside the medical operating theater (4) in which the observation cameras (56) are installed.
